# EUROPEAN PATENT APPLICATION

(11) **EP 0 904 746 A2**
(43) Date of publication of application: **31.03.1999**
(21) Application number: 98307873.4
(22) Date of filing: 29.09.1998
(51) Int. Cl.: A61F 2/06

(54) **A tool for inserting a stent into a catheter**

(30) Priority: 30.09.1997 US 941339
(71) Applicant: Cordis Corporation, Miami Lakes Florida 33014 (US)
(72) Inventor: Pai, Suresh Subraya, Sunnyvale, CA 94086 (US); Scheidt, Karl K., Pembroke Pines, Florida 33024 (US)
(74) Representative: Mercer, Christopher Paul

(57) **Abstract**

In accordance with the present invention there is provided a stent insertion tool for insertion into a catheter prior to insertion of a balloon catheter having a stent crimped on the balloon. The tool is a rigid tubular member having a distal end for insertion into a catheter, a proximal for end for remaining outside of the catheter, and a longitudinal axis extending therebetween. The tool has a substantially smooth inner surface. The tool further includes a slot, having a predetermined width, extending along the longitudinal axis of the tubular member from its distal end to its proximal end.

## Description

### BACKGROUND OF THE INVENTION

Catheters, cannulas, or catheter sheath introducers, having hemostasis valves which are mounted on a housing on the end of a tube are well known in the art. An example of a catheter sheath introducer is given in commonly assigned U.S. Patent 4,895,565 issued to Hillstead on January 23, 1990 which is hereby incorporated herein by reference. These catheters have a distal end for insertion into the patient and a proximal end which remains external of the patient. Such catheters are used to facilitate the introduction of other catheters and guidewires into the vascular system ofa patient, while minimizing injury to the patient at the access site. For some procedures, such as percutaneous transluminal angioplasty, one or more catheters are inserted into and removed from the patient repeatedly. The presence of the catheter sheath introducer causes the trauma to the body to be limited to only one catheter entering at the body access site. All other catheters and guidewires pass through the catheter introducer, and thus are not traumatic to the body at the access site.

Catheter sheath introducers typically have a hemostasis valve located within a housing at the proximal end. The valve can be made from a slit elastomeric partition or membrane. The valve is designed to seal against leakage of blood, as catheters and guidewires of varying diameters are passed therethrough.

Recently, procedures such as balloon angioplasty have been followed by an implantation of a stent at the site of the previous angioplasty. Examples of stents and stenting procedures can be found in U.S. Patents 4,733,665 issued to Palmaz on March 29, 1988 and 5,019,090 issued to Pinchuck on May 28, 1991, both of which are hereby incorporated herein by reference. Such stents are usually crimped onto a deflated balloon catheter, inserted through a catheter sheath introducer and guided to an artery or other vessel. Once at the target site within the vessel the balloon is inflated so as to expand and implant the stent.

Accurate placing of the stent on the balloon is required so that full expansion of the stent is achieved. In addition, because the stent is typically made from metal and the balloon is made from polymers, careful handling of the device is necessary to prevent the stent from puncturing the balloon. It is for these reasons that there has been some difficulty in inserting a balloon catheter, having a stent mounted thereon, through the hemostasis valve of a catheter sheath introducer. The flaps or slits of the valve may make sufficient contact with the stent to either cause the stent to move with respect to the balloon or otherwise damage the balloon or stent itself. Therefore, there has been a need to provide a device which will ensure safe passage of a balloon catheter stent combination through the hemostasis valve of a catheter sheath introducer. There has also been a desire to provide such a device which can be readily removed from the catheter sheath introducer and the balloon catheter once the stent crosses the valve so that the valve can close in order to minimize back bleeding. The present invention fulfills such needs.

### SUMMARY OF THE INVENTION

In accordance with the present invention there is provided an insertion tool for inserting medical devices into the human vasculature. Preferably, the tool is designed to be used with a tubular catheter, such as a catheter sheath introducer, having a lumen of a predetermined diameter. Such catheters typically have a distal end for insertion into a vessel, a proximal end for remaining outside of the vessel, and a longitudinal axis extending therebetween. The proximal end of the catheter typically has a valve attached thereto, which opens to allow passage of medical devices into the lumen of the catheter so they can then pass into human vessels.

The insertion tool is a tubular member made from a material which is preferably more rigid than the material the catheter tube and valve are made from. The tubular member has a distal end for insertion into a catheter, a proximal end for remaining outside of the catheter, and a longitudinal axis extending therebetween. The tool preferably has a substantially smooth inner surface. The tool further includes a slot extending along its longitudinal axis from its distal end to its proximal end. In one preferred embodiment, the slot has a predetermined width which is capable of being manually enlarged.

Also, in accordance with the present invention, there is provided a method for inserting a medical device into a human vessel using the above described insertion tool and catheter. The method first involves inserting a tubular catheter into a vessel. The catheter is preferably a catheter sheath introducer having a lumen of a predetermined diameter, a distal end for insertion into a vessel, a proximal end for remaining outside of the vessel, a longitudinal axis extending therebetween, and a valve attached to the proximal end which opens to allow passage of medical devices into the lumen.

The method also includes inserting an insertion tool through the valve of the catheter and into its lumen. The insertion tool is a rigid tubular member having a distal end for insertion into a catheter, a proximal end for remaining outside of the catheter, and a longitudinal axis extending therebetween. The insertion tool further includes a slot extending along the longitudinal axis from the distal end to the proximal end.

The method also includes inserting an interventional catheter, such as a balloon catheter, into the insertion tool. The interventional catheter has a distal end carrying a medical device, such as a stent, and a proximal end which extends proximal to the proximal end of the insertion tool. Thereafter, the method involves removing the insertion tool from the catheter and sliding the interventional catheter through the slot so as to also remove the insertion tool from engagement with the interventional catheter.

### BRIEF DESCRIPTION OF THE DRAWINGS

While the specification concludes with claims which particularly point out and distinctly claim the subject matter forming the present invention, it is believed that the invention will be better understood from the following description of the preferred embodiment taken in conjunction with the accompanying drawings wherein:

Figure 1 is a perspective view of a catheter introducer, insertion tool and interventional catheter in accordance with the present invention, wherein the components are shown separately.

Figure 2 is a view similar to that of Figure 1 but showing the interventional catheter and the medical device inserted within the insertion tool.

Figure 3 is a view similar to that of Figure 2 but showing the insertion tool, along with the interventional catheter and medical device, inserted within the catheter.

Figure 4 is a view similar to that of Figure 3 but showing how the insertion tool can be removed from engagement with the catheter and interventional catheter.

Figure 5 is a simplified cross-sectional view of proximal end of catheter 1 taken along line 5-5.

Figure 6 is a simplified enlarged view of the catheter 1 and insertion 30 in accordance with the present invention.

Figure 7 is similar to that of Figure 6 but showing the insertion tool inserted within the catheter.

### DETAILED DESCRIPTION OF THE INVENTION

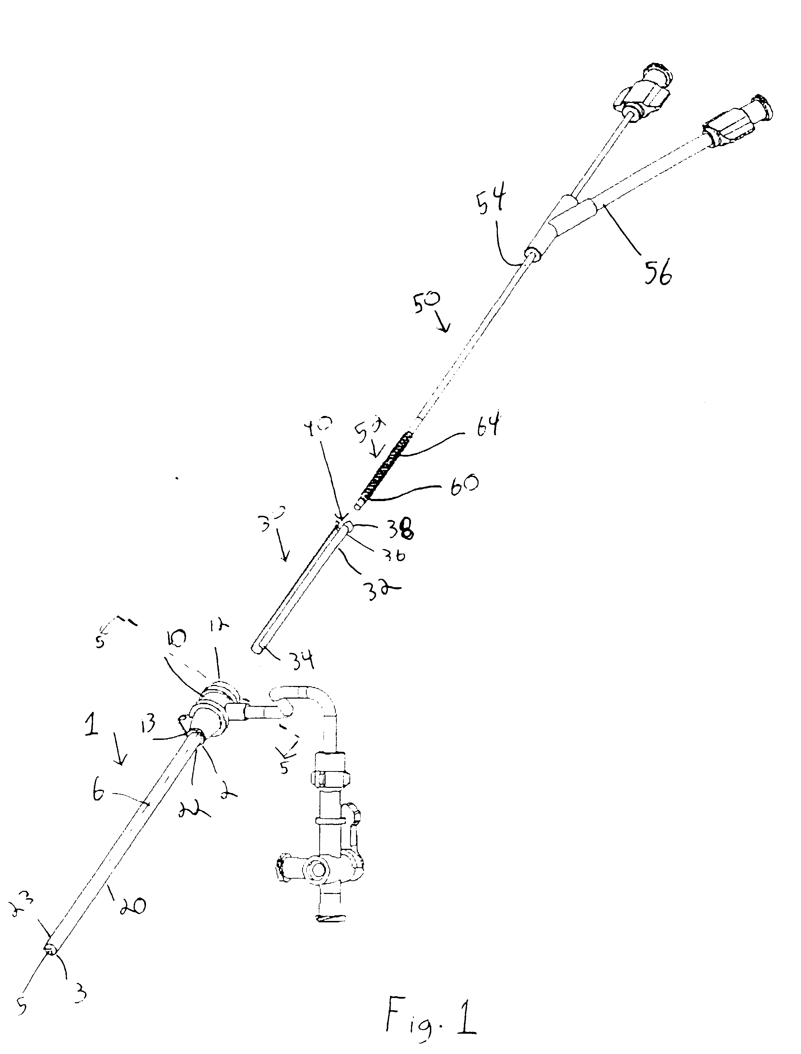

Referring to the drawings wherein like numerals indicate the same elements throughout the views, there is shown in Figures 1 a tubular catheter or catheter sheath introducer 1. Catheter 1 is designed to be inserted into the vessel of a patient and serve as an entrance site for other catheters, guidewires or the like. Catheter 1 includes a proximal end 2, a distal end 3 and a longitudinal axis 6 extending therebetween. Proximal end 2 has a rigid housing or hub 10 attached thereto. Housing 10 has a proximal end 12 and a distal end 13. Catheter 1 also has a flexible tube or cannula 20 extending between proximal end 2 and distal end 3. Tube 20 has a proximal end 22 and a distal end 23. Preferably distal end 23 of tube 20 has two tapered portions on the outside diameter (not shown). This design is set forth in U.S. patent 5,417,665 issued to De La Mata et al., which is hereby incorporated herein by reference. Catheter 1 further includes a lumen 5, having a predetermined diameter, extending through the catheter parallel to its longitudinal axis 6. The housing 10 has an annular valve 15 at its proximal end 12 for access to the lumen 5 of catheter 1.

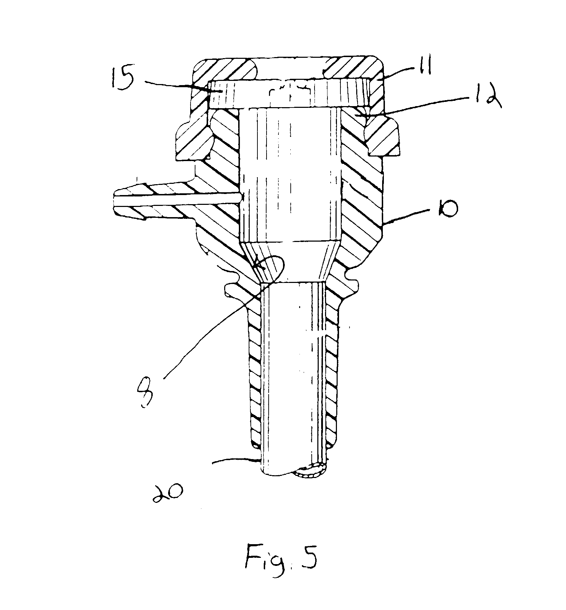

As seen from Figure 5, the housing 10 has an end cap 11, attached to its proximal end, and annular valve 15. Valve 15 preferably comprises an elastomer having 4-6 slits extending through the partition at an angle. The elastomer preferably has from about 5 to about 20 percent by weight ofa lubricity enhancing additive such as bismuth oxychloride, polytetrafluoroethylene, titanium dioxide, graphite, polyethylene wax or the like. The end cap can have a compression ring (not shown) on its interior for causing the partition to bow upwardly which enhances the sealing ofthe slits. A preferred description ofthe valve is given in U.S. patent 5,453,095 and pending U.S. patent application Serial No. 08/275,828 "Catheter Hemostasis Valve" filed on July 15, 1994, both of which are hereby incorporated herein by reference.

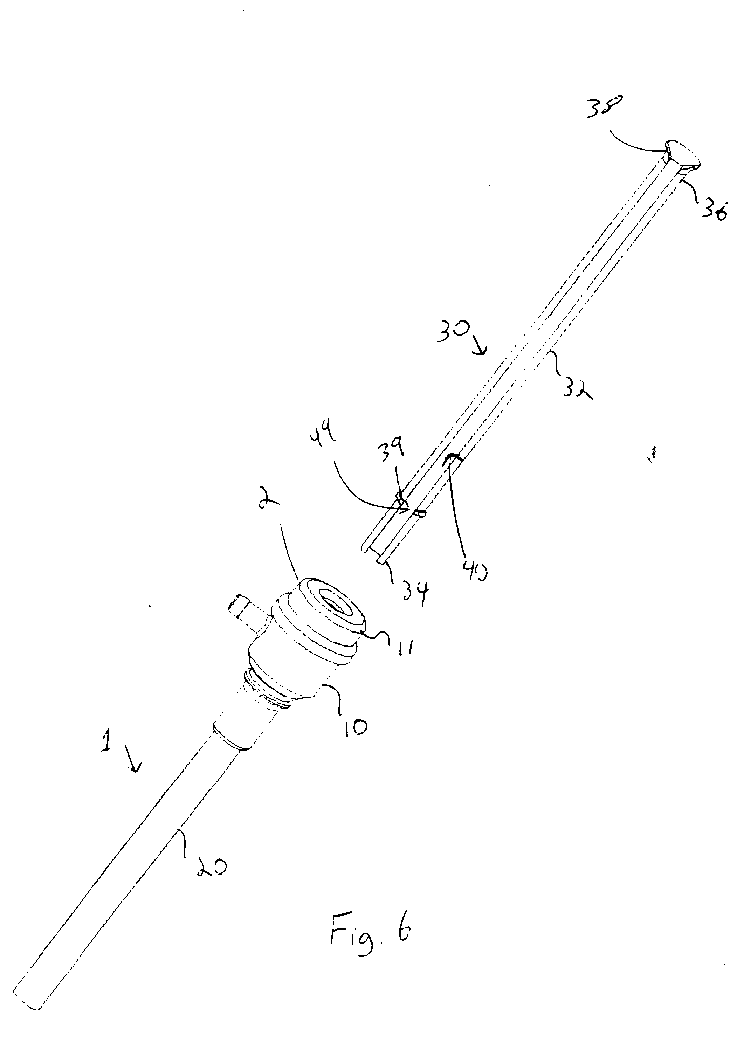

By referring to Figure 6, there is shown an insertion tool 30 made in accordance with the present invention. Tool 30 includes a tubular member 32, which is preferably made from a material which is more rigid than what tube 20 and valve 15 are made from. Preferable materials should be rigid, substantially non-compliant, but still manually pliable. Such materials include stainless steel, high density polyethylene, Nitinol, peek and polyamide. Tubular member 32 has a distal end 34, a proximal end 36 and a longitudinal axis extending therebetween. Distal end 34 is designed for insertion into the proximal end 2 of catheter I and through valve 15. Proximal end 36 preferably has outwardly flared edge 38. Member 32 preferably has a substantially smooth inner surface. Tool 30 further includes a slot 40 extending along the longitudinal axis of tubular member 32 from distal end 34 to proximal end 36.

Also shown in Figure 1, whose function is described in greater detail below, is an interventional catheter, shown in the figures as balloon catheter 50. Balloon catheter 50 has a distal end 52 having an inflatable balloon 60 disposed thereon. Such balloon catheters are described in U.S. Patent 5,304,197 issued to Pinchuck et al. on April 19, 1994, which is hereby incorporated herein by reference. Balloon 60 carries a medical device, which is shown as being stent 64 in the figures. Stent 64 has been crimped onto the balloon 60 in a typical manner which is well known to those of ordinary skill in the art. Balloon catheter 50 also has a proximal end 54. Proximal end 54 has what is known to those of ordinary skill in the art as a Y-connector 56 attached thereto. As will be appreciated by those skilled in the art a steerable guidewire is typically used in these types of procedures. An example of such a guidewire is given in U.S. Patent 5,267,574 issued to Viera et al. on December 7, 1993, which is hereby incorporated herein by reference. Such guidewires are inserted through a lumen in the balloon catheter and help guide and steer the catheter to the target site.

Many features of insertion tool can best be explained by describing a typical procedure in which it is used. The physician would first insert tubular catheter 1 into the vessel of the patient, such as the femoral artery, using any number of means well known to those of ordinary skill in the art. Proximal end 2 of the catheter and the housing 10 with valve 15 remain outside of the patient. The valve helps to prevent back bleeding but still allows other catheters and devices to be passed therethrough and into the vessel.

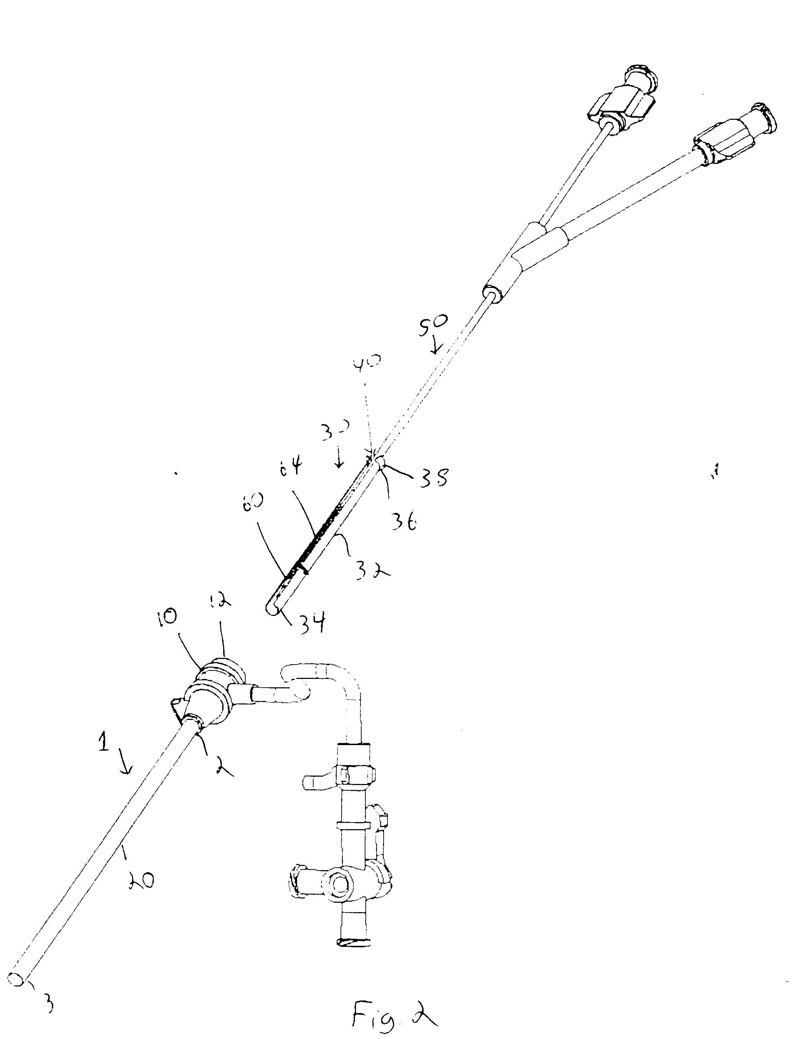

As seen from Figure 2, once the catheter 1 is in place within the vessel, the physician would then insert the balloon 60 and stent 64 into the insertion tool 30. Outwardly flared ends 38 help to ensure that the stent enters the insertion tool without snagging or otherwise being caught up on the proximal end 36. Preferably the entire length of the balloon 60 and stent 64 are completely inserted within and surrounded by tubular member 32. As seen from the figures, the proximal end 54 of the balloon catheter 50 is proximal to proximal end 36 of member 32. While the inner diameter of tubular member 30 is preferably slightly larger than the outside diameter of the crimped stent on the balloon, tube 30 should be made from a rigid, substantially non-compliant material, such as stainless steel, so that when the balloon/stent combination is inserted therein, the diameter of the tubular member does not expand anyway. This prevents the valve from damaging the stent/balloon combination when passing therethrough.

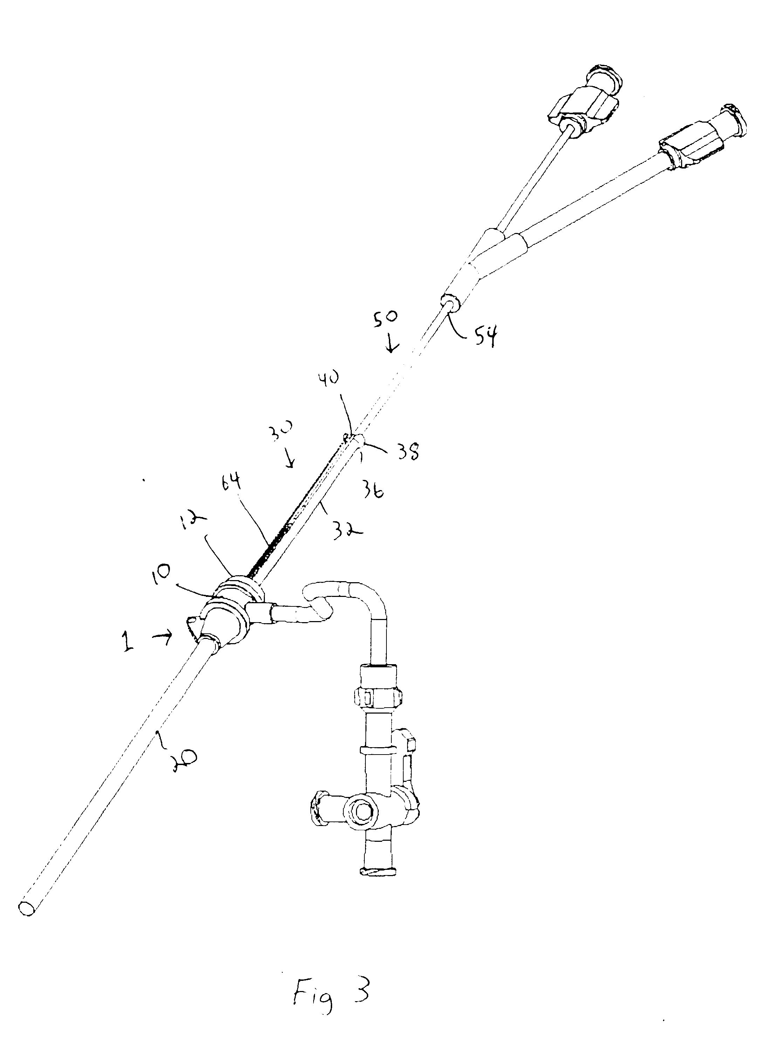

As seen from Figure 3, once the stent and the balloon are placed within the insertion tool, the insertion tool is then placed within the catheter. That is the physician passes the distal end 34 of tubular member 30 through the slitted valve 15 of catheter 1 and into lumen 5 of cannula 20. The tubular member is now holding the valve 15 open so that the stent and balloon can be placed into lumen 50 of cannula 20 while making little to no physical contact with the valve 15. As mentioned above, this prevents the valve from damaging the stent/balloon combination when passing therethrough. Once the stent passes completely through the valve, the balloon catheter can then deliver the stent to the target site within the vessel.

It should be noted that the tool 30 could be inserted into the catheter I prior to the balloon and stent being inserted into the tool which is the reverse of what is described above. The sequence of these steps are fully interchangeable within the scope of the present invention.

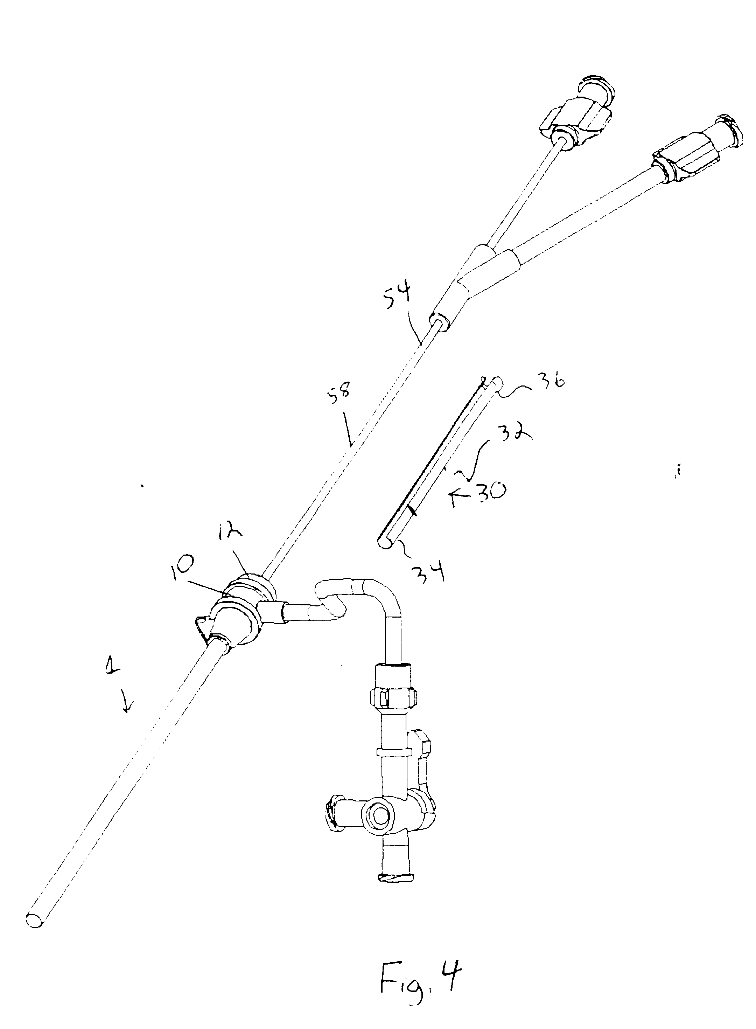

As seen from Figure 4, once the stent and balloon are passed through the valve, the insertion tool 30 can be removed from engagement with both catheter I and balloon catheter 50. This is preferable in that removing the insertion tool from catheter 1, allows the valve to close tightly around the balloon catheter and minimize back bleeding. Removing insertion tool 30 from engagement with the balloon catheter 50, removes the tool from interfering with the physicians manipulation of the balloon catheter during the procedure. In order to do this, the physician would typically first remove tool 30 from catheter I so that distal end 34 of tube 30 is proximal to proximal end 12 of housing 10. Thereafter, insertion tool 30 is removed from engagement with balloon catheter 50 by sliding the catheter through slot 40. If the width of slot 40 is greater than the diameter of balloon catheter cannula 58, then the balloon catheter should slide out easily. If not, then the slot could be manually enlarged so as to peel away the tube 30 from the balloon catheter.

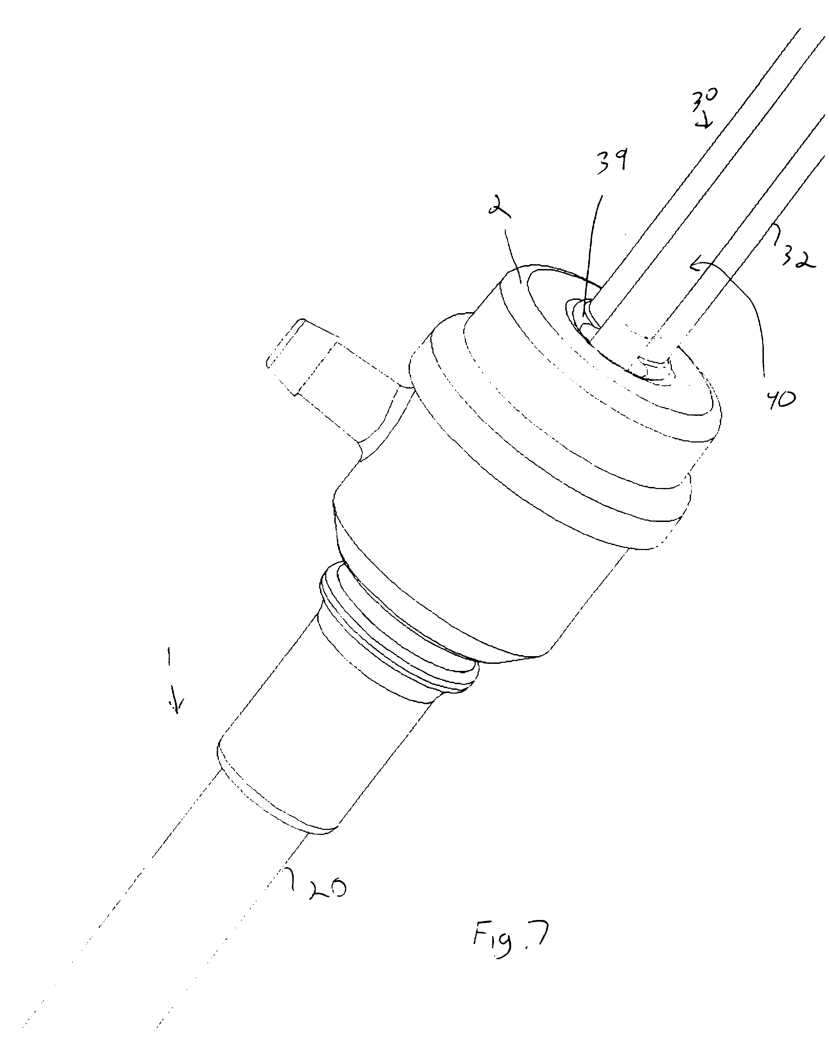

Another preferred feature of the present invention can best be described by referring back to Figure 6. As seen from that figure, insertion tool 30 preferably includes and outwardly extending bulge or band 39. Band 39 extends along the outer surface ofthe circumference of tubular member 30 and has a slot 49 which substantially corresponds to 40 on the tool. As seen from Figure 7, band 39 acts as a stop to prevent over insertion of device 30 within catheter 1. The diameter of the bulge should be greater than the opening on the catheter which gives access to the valve. Over insertion could cause unwanted interaction between the distal end 34 of device 30 and tapered interior portion 8, shown in Figure 5, of housing 10. This interaction could cause either the distal end 34 to collapse, or cause damage to the interior ofthe housing 10.

Although particular embodiments of the present invention have been shown and described, modification may be made to the catheter without departing from the spirit and scope of the present invention. The terms used in describing the invention are used in their descriptive sense and not as terms of limitations.

## Claims

1. A tool for aiding the insertion of medical devices into catheters for eventual placement within human vessels, said tool to be inserted into a cathether prior to insertion of said medical device within said vessel, said tool comprising a) a rigid tubular member, said member having a distal end for insertion into a catheter, a proximal for remaining outside of the catheter, and a longitudinal axis extending therebetween, said member having a substantially smooth inner space; and b) a slot extending along the longitudinal axis of said tubular member from said distal end to said proximal end.

2. The tool of claim 1, wherein said slot has a predetermined width.

3. The tool of claim 2, wherein said predetermined width is capable of being manually enlarged.

4. The tool of claim 2 or claim 3, wherein said predetermined width is larger than the diameter of a catheter into which it is to be inserted.

5. The tool of any one of claims 1 to 4, wherein said proximal end of said member has an outwardly flared edge.

6. The tool of any one of claims 1 to 5, further including a rigid band extending along an outer surface of a circumference of said member, said band having a slot which substantially corresponds to said slot on said tool.

7. The tool of any one of claims 1 to 6, wherein said member is made from stainless steel.

8. A device for inserting a medical device into the human vasculature, said device comprising;
a) a catheter comprising a tube having a lumen of a predetermined diameter, said catheter having a distal end for insertion into a vessel, a proximal end for remaining outside of the vessel, and a longitudinal axis extending therebetween, said proximal end of said catheter having a valve attached thereto, said valve being openable to allow passage of said medical device into said lumen; and
b) an insertion tool as defined in any one of claims 1 to 7.

9. The device of claim 8, further including an interventional catheter, having distal and proximal ends, said distal end of said interventional catheter being designed to be inserted through said insertion tool after said insertion tool is inserted though said lumen of said catheter, said distal end of said interventional catheter having a medical device disposed thereon.

10. The device according to claim 9, wherein said interventional catheter comprises a balloon catheter.
